# EUROPÄISCHE PATENTSCHRIFT

(11) **EP 2 522 289 B1**
(45) Veröffentlichungstag und Bekanntmachung des Hinweises auf die Patenterteilung: **18.02.2015**
(21) Anmeldenummer: 12169571.2
(22) Anmeldetag: 22.04.2005
(51) Int. Cl.: A61B 17/88, A61F 2/30, G09B 23/30

(54) **Dreidimensionales Modell eines menschlichen Kinderschädels in Lebensgrösse sowie Verfahren unter Verwendung des Modells**
Three-dimensional life size model of a human child's skull and method using the model
Maquette tridimensionnelle d'un crâne enfant humain grandeur nature et procédé d'utilisation de cette maquette

(30) Priorität: 23.04.2004 DE 102004020020
(43) Veröffentlichungstag der Anmeldung: 14.11.2012
(62) Teilanmeldung aus: 05737400.1
(73) Patentinhaber: IBB Technologie-Entwicklungs-Fonds GmbH & Co. KG (TEF)., 10719 Berlin (DE)
(72) Erfinder: Haberl, Hannes, 10719 Berlin (DE)
(74) Vertreter: Tegethoff, Sebastian

(56) Entgegenhaltungen:
- US-A- 3 009 265
- US-A- 5 156 777
- US-A- 5 397 361
- US-A- 6 112 109

## Beschreibung

Die Erfindung betrifft ein dreidimensionales Modell eines menschlichen Kinderschädels in Lebensgröße, ein Kit, umfassend mehrere solcher Modelle sowie Verfahren unter Verwendung des Modells.

Die Erforschung der Korrekturmöglichkeiten genetischer Störungen des Schädelwachstums auf zellulärer Ebene hat bisher nicht zu praktisch verwendbaren Ergebnissen geführt. Patienten mit prämaturen Schädelnahtsynostosen (Inzidenz 1:1000) sind deshalb weiterhin auf eine operative Korrektur angewiesen um ihr Aussehen zu verbessern oder das Risiko eines funktionellen Schadens zu senken.

Die schnelle Zunahme des Hirnvolumens in den ersten Lebensmonaten spricht für eine möglichst frühe Durchführung des notwendigen umfangreichen Eingriffes. Das im Sinne einer Risikoabwägung günstigste Operationsalter wird zwischen 4 und 12 Monaten angegeben. Der erforderliche Eingriff ist ausgedehnt und gemessen am Lebensalter mit einigen Risiken behaftet. Insbesondere der relativ hohe Blutverlust läßt eine Vereinfachung und Verkürzung der Operationszeit besonders wünschenswert erscheinen

Operationstechnisch haben sich weltweit die von Paul Tessier (Tessier P. Total facial osteotomy. Crouzon's syndrome, Apert's syndrome: oxycephaly, scaphocephaly, turricephaly. Ann Chir Plast 1967; 12(4): 273-86) vorgeschlagenen Methoden der Knochenfragmentierung, - umformung und Reposition durchgesetzt. Operationstechnik und Zeitpunkt wurden in der Folgezeit von verschiedenen Autoren variiert (Renier D, Lajeunie E, Arnaud E, Marchac D. Management of craniosynostoses. Childs Nerv Syst 2000; 16(10-11):645-58). Im Laufe der letzten 30 Jahre konnte so eine gewisse Standardisierung des tradierten technischmethodischen Vorgehens beobachtet werden.

Einige wichtige und zeitraubende Aspekte des operativen Vorgehens haben allerdings bisher keine richtungsweisende Diskussion erfahren:
1. Die Kriterien für ein exaktes Form-"Ziel" fehlen. Die wichtigste und oft einzige Operationsindikation - die möglichst perfekte Annäherung an die fiktive "gesunde" Schädelform des Patienten - bleibt ohne Planungskriterien dem Formgefühl des Operateurs bzw. seinen willkürlichen ästhetischen Kriterien überlassen. Die Operationszeit kann durch immer wieder erforderliche Korrekturen im Sinne eines Herantastens an die optimale Form verlängert werden. Eine objektive Erfolgskontrolle ist wegen der individuellen Ausformung nahezu ausgeschlossen.
2. Formwahl und Realisierung im Bereich unterschiedlich stark gekrümmter Kalottenabschnitte, etwa im Stirnbereich, besitzen aufgrund der Materialsteifigkeit auch für den trainierten Operateur einen hohen Schwierigkeitsgrad.

Sie sind langwierig und zeigen nicht seiten in zumindest einer Ebene suboptimale Ergebnisse.

Das Patent US 5,156,777 betrifft die Herstellung eines Schädel-Implantates. Dazu wird ein Schädel dreidimensional gescannt und davon ein Wachsabguss hergestellt. Aus diesem Wachsabguss können dann einzelne Teile gezielt ausgeschnitten und wiederum abgegossen werden. Es wird somit die Erstellung von Teilen eines bereits existierenden Schädels offenbart. Es geht jedoch nicht um Planungskriterien bei der Erstellung von Schädelformen von Patienten.

In ähnlicher Weise beschäftigt sich auch das Patent US 6,112,109 mit der Erstellung von Prothesen unter Ausnutzung von Scan-Daten, so dass mit einem 3D-Modell ein genaues Abbild erstellt wird.

In dem Patent US 3,009,265 geht es um ein Schädelmodell, welches aus mehreren Komponenten besteht, zum Einsatz in der Lehre oder in der Kunst. Es geht nicht um Planungskriterien bei der Erstellung von Schädelformen von Patienten.

Das Patent US 5,397,361 beschäftigt sich mit Kranialplatten und somit mit Teilen des Hirnschädels, die modelliert und implantiert werden sollen. Es geht nicht um dreidimensionale Modelle von Kinderschädeln.

Dementsprechend war es Aufgabe der vorliegenden Erfindung, Zielkriterien für eine Behandlung von Cranialsynostosen bereitzustellen. Es war eine weitere Aufgabe der Erfindung, die operationstechnischen Probleme der Schädelumformung bei angeborenen oder erworbenen Schädeldeformitäten zu lösen.

Eine weitere Aufgabe der vorliegenden Erfindung war es, ein Instrumentarium zur Verbesserung und Vereinfachung operativer Schädelrekonstruktionen im ersten Lebensjahr bereitzustellen.

Diese Aufgaben werden gelöst durch ein dreidimensionales Modell eines menschlichen Kinderschädels in Lebensgröße, umfassend einen Stirnteil (Frontalmodul), einen Scheitelteil (Parietalmodul) und einen Hinterhauptteil (Occipitalmodul), dadurch gekennzeichnet, daß es sterilisierbar ist.

Der Begriff "sterilisierbar", wie hierin verwendet, soll bedeuten, daß das Modell in der Lage ist, Temperaturen und Drücken und/oder Gaseinwirkungen zu widerstehen, wie sie üblicherweise beim Autoklavieren oder Sterilisieren von Operationsbesteck, medizinischem Gerät zum Einsatz am und im Patienten etc. verwendet werden, ohne daß das Modell durch einen solchen Sterilisierungsvorgang beschädigt, insbesondere deformiert würde. Bevorzugt ist das Modell aus einem entsprechenden hierfür geeigneten Material, bspw. Stahl, Titan oder Kunststoff.

In einer Ausführungsform ist vorgesehen, daß in dem Modell mindestens zwei Teile, ausgewählt aus Stirnteil, Scheitelteil und Hinterhauptteil, miteinander verbunden sind, wobei bevorzugt ist, daß der Scheitelteil und Hinterhauptteil miteinander zu einem Scheitel- und Hinterhauptteil (Parieto-Occipitalmodul) verbunden sind.

In einer Ausführungsform ist vorgesehen, daß das erfindungsgemäße dreidimensionale Modell zusätzlich einen Basisteil (Basismodul) umfaßt, auf dem Stirnteil, Scheitelteil und Hinterhauptteil angeordnet sind.

In einer Ausführungsform ist vorgesehen, daß das erfindungsgemäße dreidimensionale Modell einen durchschnittlichen Kinderschädel darstellt, wobei der Durchschnitt auf eine normal verteilte Population von Kindern bezogen ist, die 12-20 Wochen alt sind.

In einer anderen Ausführungsform ist vorgesehen, daß das erfindungsgemäße dreidimensionale Modell einen durchschnittlichen Kinderschädel darstellt, wobei der Durchschnitt auf eine normal verteilte Population von Kindern bezogen ist, die 24-32 Wochen alt sind.

Es wird bevorzugt, daß das erfindungsgemäße Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser und horizontaler Krümmungsradius des Occipitalmoduls, einen durchschnittlichen Kinderschädel darstellt.

In einer Ausführungsform ist vorgesehen, daß das erfindungsgemäße dreidimensionale Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser und horizontaler Krümmungsradius des Occipitalmoduls, auf einer Perzentilenkurve liegt, die ≤ 10% ist, bezogen auf eine normal verteilte Population von Kindern im Alter von 0-1 Jahren, wobei bevorzugt ist, daß es auf einer perzentilen Kurve liegt, die 3% ≤ x% ≤ 10%, bevorzugt 3% ist.

In einer anderen Ausführungsform ist vorgesehen, daß das erfindungsgemäße dreidimensionale Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 10% < x% ≤ 30% ist, bezogen auf eine normal verteilte Population von Kindern im Alter von 0-1 Jahren.

In einer anderen Ausführungsform ist vorgesehen, daß das erfindungsgemäße dreidimensionale Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 30% < x% ≤ 70% ist, bezogen auf eine normal verteilte Population von Kindern im Alter von 0-1 Jahren, wobei bevorzugt ist, daß es auf einer Perzentilenkurve liegt, die 45% ≤ x% ≤ 55% ist, bezogen auf eine normal verteilte Population von Kindern im Alter von 0-1 Jahren, wobei besonders bevorzugt ist, daß es auf einer Perzentilenkurve liegt, die 50% ist, bezogen auf eine normal verteilte Population von Kindern im Alter von 0-1 Jahren.

In einer Ausführungsform ist vorgesehen, daß das erfindungsgemäße dreidimensionale Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 55% < x% ≤ 70% ist, bezogen auf eine normal verteilte Population von Kindern im Alter von 0-1 Jahren.

In einer anderen Ausführungsform ist vorgesehen, daß das erfindungsgemäße dreidimensionale Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 70% < x% ≤ 90% ist, bezogen auf eine normal verteilte Population von Kindern im Alter von 0-1 Jahren.

In einer anderen Ausführungsform ist vorgesehen, daß das erfindungsgemäße dreidimensionale Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 90% < x% ≤ 97% ist, bezogen auf eine normal verteilte Population von Kindern im Alter von 0-1 Jahren, wobei bevorzugt ist, daß es auf einer Perzentilenkurve liegt, die 97% ist, bezogen auf eine normal verteilte Population von Kindern im Alter von 0-1 Jahren.

In einer Ausführungsform ist vorgesehen, daß das erfindungsgemäße dreidimensionale Modell auf der jeweiligen Perzentilenkurve im Lebensalterbereich von Kindern im Alter von 12-20 Wochen liegt.

In einer anderen Ausführungsform ist vorgesehen, daß das erfindungsgemäße dreidimensionale Modell auf der jeweiligen Perzentilenkurve im Lebensalterbereich von Kindern im Alter von 24-32 Wochen liegt.

Es ist bevorzugt, daß das erfindungsgemäße dreidimensionale Modell in seinen äußeren Dimensionen der Knochenstärke eines menschlichen Kinderschädels entsprechend um 1-5 mm reduziert ist. Der entsprechende Wert kann patientenabhängig innerhalb des angegebenen Bereichs variieren.

In einer Ausführungsform ist vorgesehen, daß das erfindungsgemäße dreidimensionale Modell eine Formschale umfaßt, die, abnehmbar, einem inneren Stützelement aufliegt, wobei bevorzugt ist, daß die Formschale den Stirnteil (Frontalmodul), den Scheitelteil (Parietalmodul) und den Hinterhauptteil (Occipitalmodul), die, besonders bevorzugt, untereinander verbunden sind, umfaßt.

In einer Ausführungsform ist vorgesehen, daß das innere Stützelement ein Gestell oder Rahmen ist, auf dem die Formschale aufliegt.

In einer Ausführungsform ist vorgesehen, daß die äußere Oberfläche der Formschale Aussparungen zur Aufnahme von Stabilisierungselementen enthält, wobei bevorzugt ist, daß die Aussparungen Öffnungen in der Formschale sind, bevorzugt streifen- und/oder punktförmige Öffnungen.

In einer Ausführungsform ist vorgesehen, daß die Aussparungen auf der äußeren Oberfläche der Formschale in regelmäßigen Abständen zueinander angeordnet sind.

In einer Ausführungsform ist vorgesehen, daß die Formschale Aussparungen aufweist, die eine Verbindung von auf die äußere Oberfläche der Formschale und/oder in die Aussparungen aufgebrachten Stabilisierungselementen mit ebenfalls von außen auf die Formschale aufgebrachten Knochenplatten ermöglichen, wobei bevorzugt ist, daß die Verbindung mittels Verschraubung oder Vernietung stattfindet, und wobei besonders bevorzugt ist, daß die zuletztgenannten Aussparungen ganz oder teilweise mit den davorgenannten Aussparungen identisch sind.

Die Aufgaben der Erfindung werden auch gelöst durch ein dreidimensionales Modell gemäß der vorliegenden Erfindung zur Verwendung in einem Verfahren zur operativen Behandlung einer Craniosynostose.

Weiterhin werden die Aufgaben der Erfindung gelöst durch einen Kit, umfassend mindestens ein, bevorzugt mindestens zwei, bevorzugt mindestens drei unterschiedliche Modelle gemäß der vorliegenden Erfindung, wobei bevorzugt ist, daß das Kit fünf, bevorzugt sechs unterschiedliche Modelle gemäß der vorliegenden Erfindung umfaßt.

In einer Ausführungsform ist vorgesehen, daß der Kit weiterhin ein oder mehrere Teile umfaßt, ausgewählt aus der Gruppe:
- resorbierbare Stabilisierungselemente, insbesondere Platten oder Streifen, die zum Stabilisieren von Schädelknochenfragmenten geeignet sind,
- resorbierbare Schrauben und/oder Nieten zum Fixieren von Schädelknochenfragmenten auf den Stabilisierungselementen,
- Werkzeug zum Einbringen und/oder Entfernen der Schrauben oder Nieten, insbesondere Schraubendreher und Schraubenentferner.

Dabei ist bevorzugt, daß die resorbierbaren Stabilisierungselemente in erhitztem Zustand formbar sind, wobei besonders bevorzugt ist, daß die resorbierbaren Stabilisierungselemente resorbierbare Milchsäureplatten oder -streifen sind.

Solche resorbierbaren Milchsäureplatten oder -streifen sind kommerziell erhältlich.

In einer Ausführungsform ist vorgesehen, daß die Platten oder Streifen physiologisch akzeptable Dicke, Breite und Länge aufweisen, wobei ein Kit einheitlich große Platten und Streifen oder unterschiedlich große Platten und Streifen enthält.

Die Dimensionen der Platten und Streifen sind bevorzugt patientenabhängig auszuwählen. Dabei ist vorgesehen, daß der Kit bevorzugt unterschiedlich große Platten und/oder Streifen enthält, um den jeweiligen individuellen Gegebenheiten des jeweiligen Operationssitus Rechnung tragen zu können.

Die Aufgaben der Erfindung werden auch durch einen erfindungsgemäßen Kit zur Verwendung in einem Verfahren zur operativen Behandlung einer Craniosynostose gelöst.

Weiterhin werden die Aufgaben der Erfindung gelöst durch ein Verfahren zur operativen Behandlung einer Craniosynostose bei einem Patienten, umfassend die Schritte:
- Steriles Bereitstellen eines dem Patienten nach Vergleichmessung ähnlichsten dreidimensionalen Modells gemäß der vorliegenden Erfindung,
- Auflegen von in erhitztem Zustand formbaren Stabilisierungselementen, bevorzugt resorbierbaren Milchsäureplatten, auf das Modell und Aushärtung der Stabilisierungselemente,
- Entnahme eines von der Craniosynostose betroffenen, fehlgebildeten Kalottenabschnitts und Auftrennung des Kalottenabschnitts in Fragmente, bevorzugt Streifen,
- Aufformung der Fragmente des fehlgebildeten Kalottenabschnitts auf das dreidimensionale Modell durch Ausdünnung, Destabilisierung und/oder Modellierung,
- Fixierung der Fragmente auf den darunterliegenden Stabilisierungselementen, bevorzugt durch Verschraubung und/oder Vernietung, wodurch ein Implantat aus Fragmenten und Stabilisierungselementen gebildet wird,
- Implantation des Implantats durch Einpassung und Fixierung, bevorzugt Verschraubung, in dem Schädel des Patienten.

Es ist bevorzugt, daß das dreidimensionale Modell der Knochenstärke entsprechend um 1-5 mm in seinen äußeren Dimensionen reduziert ist, um eine formgerechte Implantation der umgeformten Knochenfragmente zu ermöglichen.

In einer Ausführungsform ist vorgesehen, daß zur Aufformung der Fragmente des fehlgebildeten Kalottenabschnitts auf das dreidimensionale Modell diese auf das Modell aufgebracht und unter Krafteinwirkung, bevorzugt mittels eines reversibel entfernbaren Fixiermittels, bevorzugt mittels eines elastischen Bands, in Position gehalten werden.

Es ist bevorzugt, daß zur Implantation das Implantat von dem dreidimensionalen Modell abgenommen wird.

Wesentlicher Ansatzpunkt der Erfindung ist die Umsetzung eines neuen Verfahrens der statistischen Modellbildung, das erstmals valide Daten zur Normalverteilung kindlicher Kopfformen bietet. Dabei haben die Erfinder überraschenderweise gefunden, daß durch den Einsatz eines geeigneten, individuell an den jeweiligen Patienten angepaßten dreidimensionalen Modells, welches sterilisierbar ist, gute Erfolge in planbarer und rational nachzuvollziehender Weise erzielt werden können.

Vorbereitend werden innerhalb einer bezüglich des Schädelwachstums gesunden Gruppe von Rekonstruktionen der Altersstufen 12 -20 Wochen (4 Monate) und 24-32 Wochen (8 Monate) typische Formmerkmale (Horizontale Stirnwölbung, Vertikale Stirnwölbung, Stirnbreite, Frontonasaler Winkel, frontooccipitaler Durchmesser, biparietaler Durchmesser, horizontaler Krümmungsradius der Occipitalschuppe etc.) erfaßt und analysiert. Grundsätzlich alle Meßwerte werden in Perzentilenkurven dokumentiert. Eine Perzentilenkurve, wie hierin verwendet, dient zum Vergleich eines jeweiligen Patienten mit anderen gleichaltrigen Patienten. Dabei bedeutet eine 10% Perzentile, daß 9% der gleichaltrigen Patienten hinsichtlich eines bestimmten Merkmals unter dem Wert des untersuchten Patienten, 90% der gleichaltrigen Patienten hinsichtlich desselben Merkmals über dem Wert des untersuchten Patienten liegen. Aus der Fusion der Rekonstruktionen altersidentischer Kinder wird ein virtueller Durchschnittskopf "4 Monate" bzw. "8 Monate" (~ 50% Perzentile) sowie je ein Kopf mit maximaler Formabweichungen einzelner Merkmale (Kopflänge, -breite, Stirnbreite, -wölbung, etc.) (~ 3% Perzentile bzw. ~ 97% Perzentile) berechnet und in stereolithographische Modelle umgesetzt. Dabei soll möglichst die gesamte Variationsbreite unterschiedlicher Merkmale erfaßt werden. Scheitelteil, Hinterhauptteil und Stirnteil sind miteinander verbunden. Pro Altersgruppe sind für jeden Kalottenabschnitt etwa 6 variierende Teilmodelle vorgesehen, die dann je nach gegebener Kopfform des Patienten und gewünschter Veränderung ausgesucht werden können. In der Umsetzung der Daten kann im Gegensatz zu bisher üblichen bildschirmgebunden Operationsplanungsverfahren sowohl die Erzeugung von Kosten (und Risiken) durch individuelle Bildgebung vermieden werden als auch die Präzision der Umsetzung der Formvorgabe ohne Einsatz etwa von bildabhängigen Navigationssystemen ermöglicht werden.

Dazu wird aus dem Datenpool ein Instrumentarium, basierend auf einem altersbezogenen Sortiment dreidimensionaler mehrteiliger Formmodule entwickelt. Fehlgeformte Kalottenabschnitte können dann über einem passenden erfindungsgemäßen Modell präzise umgeformt mit vorgeformten resorbierberen Stabilisierungselementen innenseitig versteift und "en bloc" reimplantiert werden).

Der Einsatz eines erfindungsgemäßen Modells oder mehrerer erfindungsgemäßer Modelle ermöglicht den Ersatz der suboptimalen Transposition von Fragmenten durch die tatsächliche mehrdimensionale Umformung unter Korrektur der Krümmungsradien auf einer formgebenden Oberfläche.

Besonderer Vorteil dieses Verfahrens ist darüber hinaus die kosmetisch günstigere innenseitige Lage der relativ dicken resorbierbaren Stabilisierungselemente.

Mit dem hier vorgestellten erfindungsgemäßen Operationskonzept bzw. den erfindungsgemäßen dreidimensionalen Modellen bzw. Kit wird dieses Ergebnis planbar und im Vergleich mit dem Ausgangsmodell der Operation jederzeit kontrollierbar. Korrekturverluste durch pathologisches Knochenwachstum können quantifiziert werden und langfristig in die Operationsvorgaben (Modellplanung) einfließen. Die Anzahl suboptimaler Ergebnisse und die Häufigkeit von Zweiteingriffen bei nichtsyndromalen Synostosen reduziert sich entsprechend.

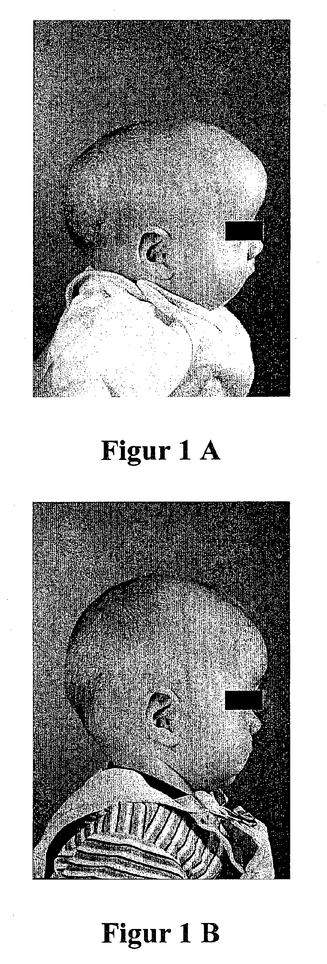

Es wird nunmehr Bezug genommen auf die Figuren, bei denen
Abbildung 1 das Beispiel eines vier Monate alten Säuglings mit Sagittalnahtsynostose vor (Fig. 1 A), und vier Wochen nach (Fig. 1 B) einer operativer Umformung zeigt,

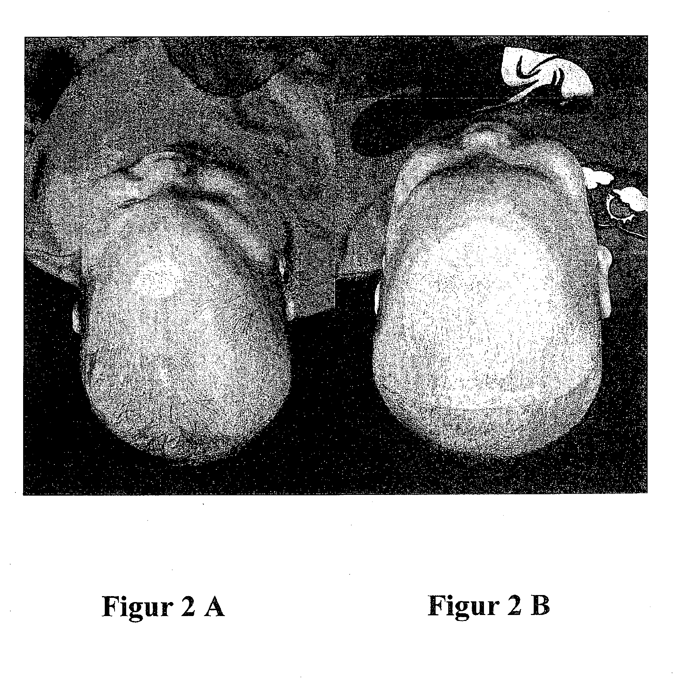

Abbildung 2 das Beispiel eines fünf Monate alten Säuglings mit Frontalnahtsynostose vor (Fig. 2 A), und vier Wochen nach (Fig. 2 B) einer operativer Umformung zeigt,

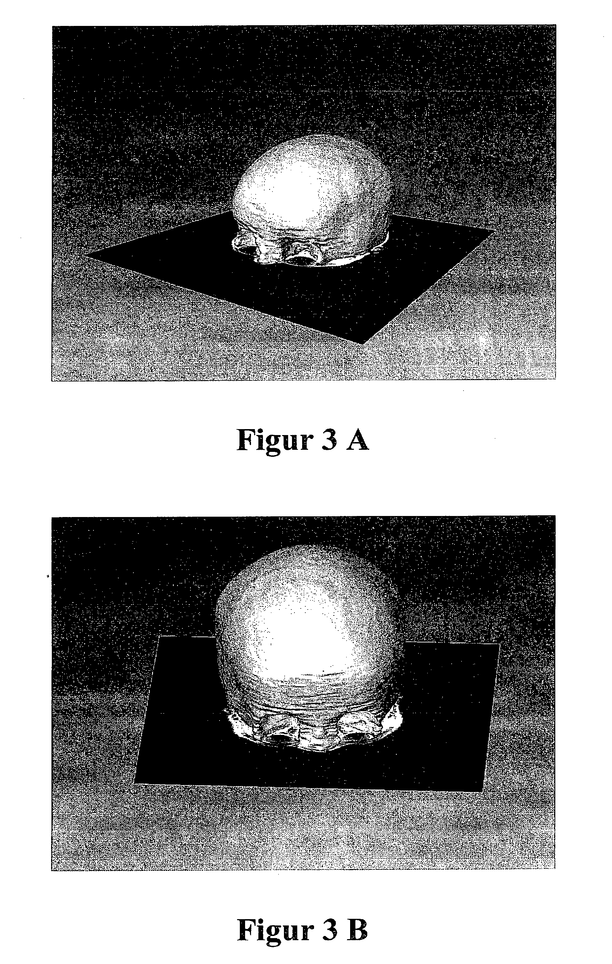

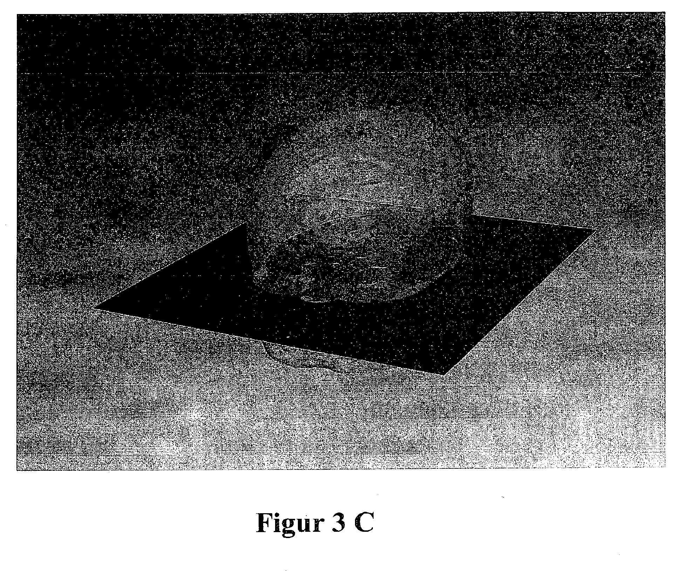

Abbildung 3 die manuelle Segmentierung einer kindlichen Schädelkalotte aus einem MRT-Datensatz (Magnetresonanztomographie) zeigt (Fig. 3A - 3C),

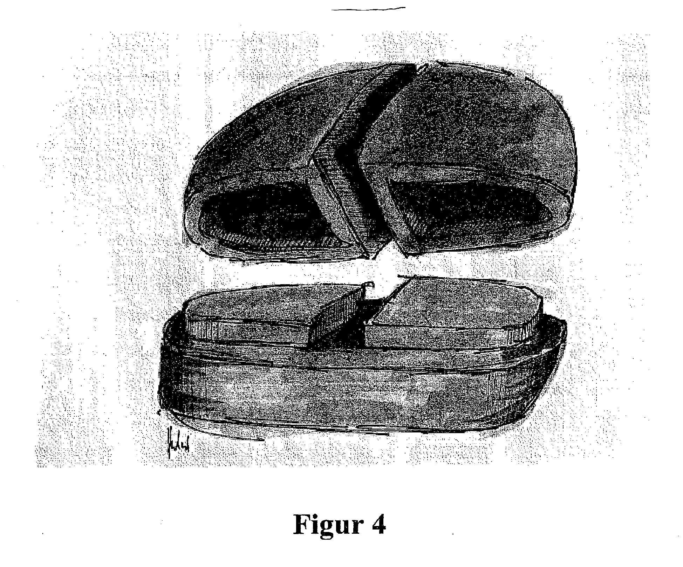

Abbildung 4 ein Beispiel eines sterilisierbaren Modells einer Schädelkalotte, bestehend aus Basis- 7, Frontal- 8 und Parieto-Occipitalmodul 9 zeigt,
Abbildung 5 die Aufformung eines wärmebiegsamen resorbierbaren Stabilisierungselementes 4 auf ein Stirnmodul 8 zeigt,
Abbildung 6 eine Aufformung des destabilisierten Knochenstreifen 10 auf das ausgehärtete Stabilisierungselement 4 über einem Stirnmodul zeigt,
Abbildung 7 ein fertig verschraubtes Implantat (Knochenstreifen auf Stabilisierungselementen) auf einem Stirnmodul zeigt,
Abbildung 8 die Reimplantation des umgeformten Stirnsegmentes in den vorbereiteten Situs zeigt,
Abbildung 9 eine auf einem inneren Stützelement 2 abnehmbar aufliegende Formschale 1 zeigt, und
Abbildung 10 die auf der Formschale aufgeformten Knochenfragmente 10 mit Stabilisierungselementen 4 zeigt, die von einem Fixierungsmittel 11, hier einem elastischen Band, in Position gehalten werden.

### Vorarbeiten zur statistischen Modellbildung

Die Erstellung von 3D Atlanten und deren Nutzung zur 3D Formanalyse ist gegenwärtig Gegenstand der Forschung in diversen Bereichen, wie z.B. der Anthropometrie, der medizinischen Bildanalyse, Ergonomiestudien. Ein 3D Atlas beschreibt dabei die mittlere Form eines Objektes und seine typischen Abweichungen innerhalb einer gegebenen Stichprobe. Die Erzeugung eines Atlas erfordert einen Formvergleich, wobei man zwischen flächen- und volumenbasierten Ansätzen unterscheidet. Das Ergebnis des Formvergleiches ermöglicht die Darstellbarkeit der Stichprobe in einem hochdimensionalen Vektorraum. In der Literatur existieren eine Reihe von Verfahren zur Extraktion relevanter statistischer Parameter, die zur Erstellung eines speziellen Atlas verwendet werden.

Zur Erstellung der Oberflächenmodelle für den 3D Atlas ist die Segmentierung (Gewebeklassifikation) von tomografischen Bilddaten erforderlich. Hierzu gibt es Softwaresysteme, die den Segmentierungsvorgang mittels manueller und semiautomatischer Werkzeuge unterstützen (Analyze, Amira, Materialise).

Ein vielversprechender Ansatz zur vollautomatischen Segmentierung basiert auf der Nutzung von à priori Wissen in Form von 3D Atlanten. Der Atlas wird zunächst auf Basis der segmentierten Daten erzeugt. Sobald er sich aber aus einer hinreichend großen Zahl an Modellen zusammensetzt, kann er auch selbst für die Segmentierung eingesetzt werden und somit den Segmentierungsvorgang vereinfachen und beschleunigen. Die ständige Erweiterung eines Atlas ist erforderlich, um mit einer bestimmten Wahrscheinlichkeit Aussagen über die generelle Form eines Objektes treffen zu können.

### Beschreibung einer bevorzugten Ausführungsform des erfindungsgemäßen Operationsverfahrens unter Verwendung von erfindungsgemäßen Modellen

Nach abgeschlossener statistischer Modellerzeugung werden für eine spezifische Altersgruppe (z.B. 4 Monate) 5-8 repräsentative Formvarianten produziert. Das dem individuellen Patienten nach Vergleichsmessung ähnlichste Modell wird steril im Operationssaal bereitgestellt.

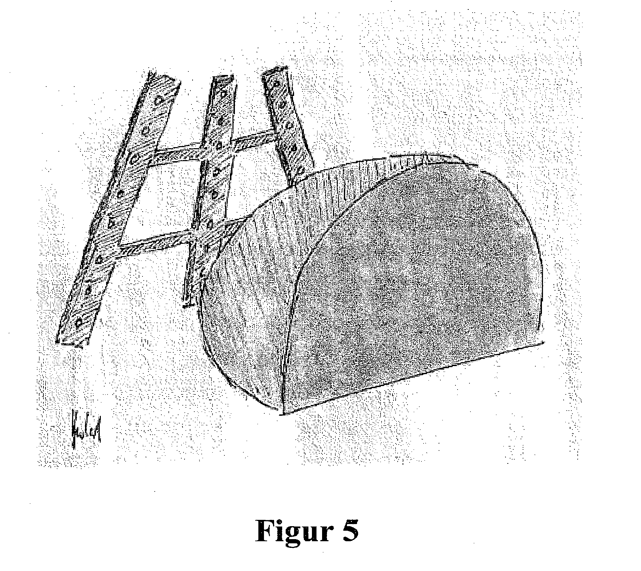

Auf das bereits um Kalottendurchmesser und Plattendurchmesser reduzierte Modell werden nun in erhitztem Zustand formbare, bevorzugt resorbierbare Stabilisierungselemente 4 (bspw. resorbierbare Milchsäureplatten) aufgelegt (Fig. 5).

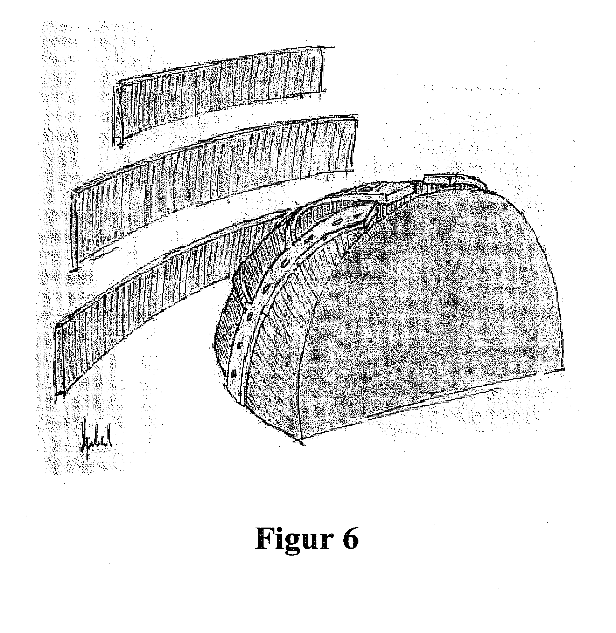

Nach Aushärtung der Formelemente wird der in Knochenstreifen zertrennte fehlgebildete Kalottenabschnitt 10 aufgeformt. Dazu werden die einzelnen Fragmente 10 ausgedünnt, destabilisiert und modelliert (Fig. 6).

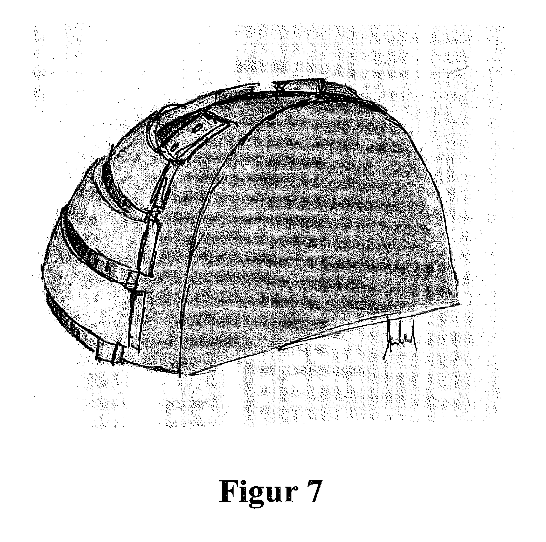

Die anliegenden Knochenstreifen 10 werden nun mit der darunterliegenden Platte aus resorbierbaren Stabilisierungselementen 4 verschraubt (Fig. 7).

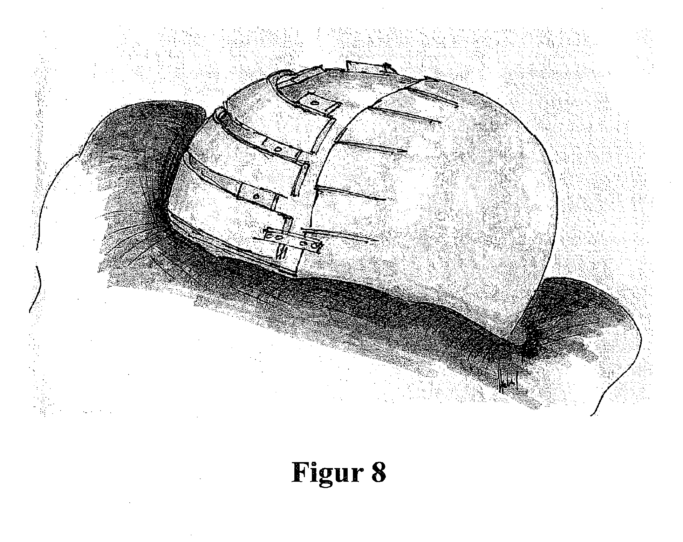

Anschließend wird der fertig umgeformte und stabilisierte Kalottenabschnitt 10 in den Situs eingebracht, durch kurze Erwärmung der Platten 4 feinangepaßt und mit der verbliebenen Kalotte 20 verschraubt (Fig. 8).

### Beschreibung einer besonders bevorzugten Ausführungsform eines erfindungsgemäßen dreidimensionales Modells

### Formschale

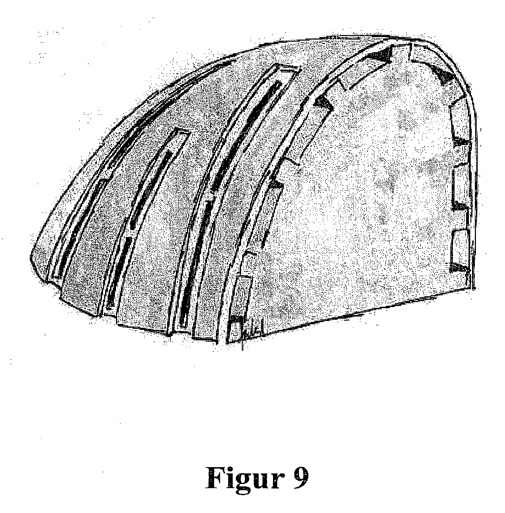

Eine bevorzugte Ausführungsform, wie in Fig. 9 gezeigt, des erfindungsgemäßen dreidimensionalen Modells umfaßt mindestens eine Formschale 1, die einem inneren Stützelement 2 abnehmbar aufliegt. Die äußeren Abmessungen der Formschale sind der Knochenstärke entsprechend, um 1-5 mm, bevorzugt um 3 mm reduziert. In die Oberfläche sind regelmäßige Einlassungen oder Aussparungen 3 zur Aufnahme verschiedener Stabilisierungselemente 4 eingearbeitet. Die Aussparungen 3 weisen schlitzförmige Öffnungen 6 auf, die einen Zugriff auf in den Aussparungen 3 eingelegten Stabilisierungselementen vom Inneren der Schale ermöglichen. Die Formschale umfaßt einen Stirnteil, einen Scheitelteil und einen Hinterhauptteil, die einzeln getrennt oder miteinander verbunden vorliegen können. In einer besonders bevorzugten Ausführungsform sind Scheitelteil und Hinterhauptteil miteinander verbunden und ergeben einen Scheitel- und Hinterhauptteil (Parieto-Occipitalmodul).

### Rumpf

Die Formschale 1 liegt - abnehmbar - einem inneren Stützelement 2 auf. Das Stützelement ist in einer bevorzugten Ausführungsform ein rahmenartiges Gerüst.

### Stabilisierungselemente

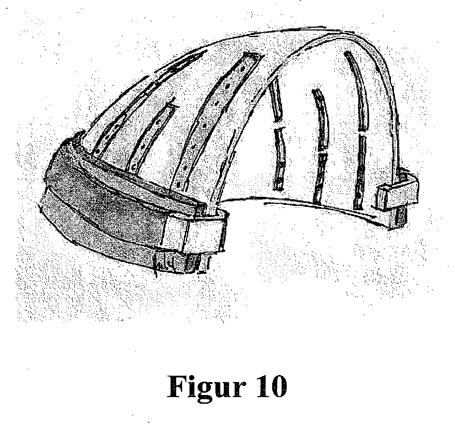

Die Einlassungen oder Aussparungen 3 der Schale werden nun, wie auch in Fig. 10 gezeigt, nach Wunsch mit erhitzt formbaren Stabilisierungselementen 4 ausgekleidet. Abweichend von der bisherigen traditionellen Operationstechnik wird nun der fehlgebildete Knochen in regelmäßige Streifen geteilt und destabilisiert. Die einzelnen Streifen werden aufgeformt und mit einem elastischen Band 5 in Position gehalten.

Anschließend wird die Schale abgenommen und durch die schlitzförmigen inneren Öffnungen 6 eine stabile Verschraubung oder Vernietung der Stabilisierungselemente mit den Knochenstreifen 10 vorgenommen.

Das fertige Implantat wird dann von der Schale genommen, fein angepasst und en bloc implantiert.

## Patentansprüche

1. Verfahren zum Erstellen eines real existierenden dreidimensionalen Modells eines Kinderschädels, **gekennzeichnet durch** die Schritte:
- Erfassen von Schädelformmerkmalen ausgewählt aus der Gruppe der Merkmale umfassend horizontale Stirnwölbung, vertikale Stirnwölbung, Stirnbreite, frontonasaler Winkel, frontooccipitaler Durchmesser, biparietaler Durchmesser, horizontaler Krümmungsradius der Occipitalschuppe, Kopflänge, -breite in einer Gruppe von bezüglich des Schädelwachstums gesunden Patienten vergleichbaren Alters und Erstellen von Perzentilenkurven aus den daraus erhobenen Messwerten,
- Berechnen eines virtuellen Durchschnittsschädels aus den Schädelformmerkmalen sowie eines Schädels mit jeweils maximal abweichender Formmerkmale aus den erstellten Perzentilenkurven,
- Umsetzen der berechneten Kinderschädel in jeweils ein real existierendes dreidimensionales Modell eines menschlichen Kinderschädels in Lebensgröße, umfassend einen Scheitelteil, einen Hinterhauptteil und einen Stirnteil, wobei mindestens zwei der genannten Teile miteinander verbunden sind.

2. Verfahren nach Anspruch 1, **dadurch gekennzeichnet, dass** der Schritt des Erfassens von Schädelformmerkmalen ferner die Analyse der Schädelformmerkmale umfasst, und wobei das Erstellen des Kinderschädels auf den erfassten und analysierten Schädelformmerkmalen basiert.

3. Verfahren nach Anspruch 1 oder 2, **dadurch gekennzeichnet, dass** Stirnteil, Scheitelteil und Hinterhauptteil abnehmbar angeordnet sind.

4. Verfahren nach einem der Ansprüche 1 bis 3, **dadurch gekennzeichnet, dass** das dreidimensionale Modell hinsichtlich eines oder mehrerer der Merkmale ausgewählt aus der Gruppe umfassend horizontale Stirnwölbung, vertikale Stirnwölbung, Stirnbreite, frontonasaler Winkel, frontooccipitaler Winkel, frontooccipitaler Durchmesser, biparietaler Durchmesser und horizontaler Krümmungsradus des Occipitalmoduls auf einer Perzentilenkurve liegt, die < 10% ist, bezogen auf eine normal verteilte Population von Kindern im Alter von 0 - 1 Jahren.

5. Verfahren nach einem Ansprüche 1 bis 3, **dadurch gekennzeichnet, dass** das dreidimensionale Modell hinsichtlich eines oder mehrerer der Merkmale ausgewählt aus der Gruppe umfassend horizontale Stirnwölbung, vertikale Stirnwölbung, Stirnbreite, frontonasaler Winkel, frontooccipitaler Winkel, frontooccipitaler Durchmesser, biparietaler Durchmesser und horizontaler Krümmungsradus des Occipitalmoduls auf einer Pertenzitilenkurve liegt, die 10% < x% < = 30% ist bezogen auf eine normal verteilte Population von Kindern im Alter von 0 bis 1 Jahren.

6. Verfahren nach einem der Ansprüche 1 bis 3, **dadurch gekennzeichnet, dass** das dreidimensionale Modell hinsichtlich eines oder mehrerer der Merkmale ausgewählt aus der Gruppe horizontale Stirnwölbung, vertikale Stirnwölbung, Stirnbreite, frontonasaler Winkel, frontooccipitaler Winkel, frontooccipitaler Durchmesser, biparietaler Durchmesser und horizontaler Krümmungsradus des Occipitalmoduls auf einer Perzentilenkurve liegt, die 30% < x% < 70% ist bezogen auf eine normal verteilte Population von Kindern im Alter von 0 bis 1 Jahren.

7. Verfahren nach einem der Ansprüche 1 bis 3, **dadurch gekennzeichnet, dass** das dreidimensionale Modell hinsichtlich eines oder mehrerer der Merkmale ausgewählt aus der Gruppe horizontale Stirnwölbung, vertikale Stirnwölbung, Stirnbreite, frontonasaler Winkel, frontooccipitaler Winkel, frontooccipitaler Durchmesser, biparietaler Durchmesser und horizontaler Krümmungsradus des Occipitalmoduls auf einer Perzentilenkurve liegt, die 70% < x% < 90% ist bezogen auf eine normal verteilte Population von Kindern im Alter von 0 bis 1 Jahren.

8. Verfahren nach einem der Ansprüche 1 bis 3, **dadurch gekennzeichnet, dass** das dreidimensionale Modell hinsichtlich eines oder mehrerer der Merkmale ausgewählt aus der Gruppe horizontale Stirnwölbung, vertikale Stirnwölbung, Stirnbreite, frontonasaler Winkel, frontooccipitaler Winkel, frontooccipitaler Durchmesser, biparietaler Durchmesser und horizontaler Krümmungsradus des Occipitalmoduls auf einer Perzentilenkurve liegt, die 90% < x% < 97% ist bezogen auf eine normal verteilte Population von Kindern im Alter von 0 bis 1 Jahren.

9. Verfahren nach einem der Ansprüche 1 bis 8, **dadurch gekennzeichnet, dass** das dreidimensionale Modell in seiner äußeren Dimension der Knochenstärke eines menschlichen Kindermodellschädels entsprechend um 1 bis 5 mm reduziert ist.

## Claims

1. A method for preparing an actually existing three-dimensional model of a child's skull, **characterized by** the steps:
- establishing skull shape characteristics, selected from the group of characteristics comprising horizontal curvature of the forehead, vertical curvature of the forehead, width of the forehead, fronto-nasal angle, fronto-occipital diameter, biparietal diameter, horizontal radius of curvature of the occipital square, length of the head, width of the head, in a group of patients of a comparable age who are healthy in terms of growth of the skull and generating percentile curves from the collected measured data,
- calculating a virtual average skull from the skull shape characteristics as well as a skull with maximum deviations of the respective shape characteristics from the generated percentile curves,
- converting the calculated children's skulls in a respective actually existing three-dimensional model of a human child's skull in life-size, comprising a parietal part, an occipital part and a frontal part, wherein at least two of said parts are connected to each other.

2. The method according to claim 1, wherein the step of establishing skull shape characteristics further comprises the analysis of the skull shape characteristics and wherein the preparing of the child's skull is based on the established and analyzed skull shape characteristics.

3. The method according to claim 1 or 2, wherein the frontal part, parietal part and occipital part are arranged detachably.

4. The method according to any one of claims 1 to 3, wherein the three-dimensional model with respect to one or several of the features selected from the group comprising horizontal curvature of the forehead, vertical curvature of the forehead, width of the forehead, fronto-nasal angle, fronto-occipital angle, fronto-occipital diameter, biparietal diameter and horizontal radius of curvature of the occipital module is located on a percentile curve representing < 10 %, relative to a normal distribution within the population of children in the age group of 0 - 1 years.

5. The method according to any one of claims 1 to 3, wherein the three-dimensional model with respect to one or several of the features selected from the group comprising horizontal curvature of the forehead, vertical curvature of the forehead, width of the forehead, fronto-nasal angle, fronto-occipital angle, fronto-occipital diameter, biparietal diameter and horizontal radius of curvature of the occipital module is located on a percentile curve representing 10 % < x % < = 30 %, relative to a normal distribution within the population of children in the age group of 0 - 1 years.

6. The method according to any one of claims 1 to 3, wherein the three-dimensional model with respect to one or several of the features selected from the group comprising horizontal curvature of the forehead, vertical curvature of the forehead, width of the forehead, fronto-nasal angle, fronto-occipital angle, fronto-occipital diameter, biparietal diameter and horizontal radius of curvature of the occipital module is located on a percentile curve representing 30 % < x % < 70 %, relative to a normal distribution within the population of children in the age group of 0 - 1 years.

7. The method according to any one of claims 1 to 3, wherein the three-dimensional model with respect to one or several of the features selected from the group comprising horizontal curvature of the forehead, vertical curvature of the forehead, width of the forehead, fronto-nasal angle, fronto-occipital angle, fronto-occipital diameter, biparietal diameter and horizontal radius of curvature of the occipital module is located on a percentile curve representing 70 % < x % < 90 %, relative to a normal distribution within the population of children in the age group of 0 - 1 years.

8. The method according to any one of claims 1 to 3, wherein the three-dimensional model with respect to one or several of the features selected from the group comprising horizontal curvature of the forehead, vertical curvature of the forehead, width of the forehead, fronto-nasal angle, fronto-occipital angle, fronto-occipital diameter, biparietal diameter and horizontal radius of curvature of the occipital module is located on a percentile curve representing 90 % < x % < 97 %, relative to a normal distribution within the population of children in the age group of 0 - 1 years.

9. The method according to any one of claims 1 to 8, wherein the exterior dimensions of the three-dimensional model are reduced by 1 to 5 mm in correspondence with the thickness of the bone of a human child's model skull.

## Revendications

1. Procédé pour réaliser un modèle tridimensionnel réel existant d'un crâne d'enfant, comprenant les étapes suivantes :
- Etablir des caractéristiques de forme de crâne, choisies dans le groupe des caractéristiques comprenant courbure de front horizontale, courbure de front verticale, largeur de front, angle fronto-nasal, diamètre fronto-occipital, diamètre bipariétal, rayon de courbure horizontale de la zone occipitale, longueur de tête, largeur de tête, dans un groupe de patients d'âge comparable et sains en terme de croissance crânienne, et réaliser de courbes en percentiles à partir des données de mesure récupérées-sélectionnées,
- Calculer un crâne moyen virtuel à partir des caractéristiques de forme de crâne ainsi que d'un crâne avec respectivement des déviations maximales des caractéristiques de forme à partir des courbes en percentiles réalisées,
- Convertir les crânes d'enfant calculés dans un modèle tridimensionnel réel existant d'un crâne d'enfant humain de taille humaine, comprenant une partie de sommet, une partie principale arrière et une partie frontale qui sont reliées les unes aux autres, dans lequel au moins deux des parties citées sont reliées l'une l'autre.

2. Procédé selon la revendication 1, **caractérisé en ce que** l'étape d'établir des caractéristiques de forme de crâne comprend en outre l'analyse des caractéristiques de forme de crâne, et dans lequel le calcul du crâne d'enfant se fonde sur les caractéristiques de forme de crâne acquises et analysées.

3. Procédé selon la revendication 1 ou 2, **caractérisé en ce que** la partie de front, la partie de sommet et la partie principale arrière sont agencées de manière amovible.

4. Procédé selon l'une des revendications 1 à 3, **caractérisé en ce que**, par rapport à une ou plusieurs caractéristique(s) choisie(s) dans le groupe comprenant courbure de front horizontale, courbure de front verticale, largeur de front, angle fronto-nasal, angle fronto-occipital, diamètre fronto-occipital, diamètre bipariétal, et rayon de courbure horizontal de module occipital, le modèle tridimensionnel se trouve sur une courbe en percentiles qui représente < 10 % par rapport à une population d'enfants normalement répartie d'âge 0-1 an.

5. Procédé selon l'une des revendications 1 à 3, caractérisé en ce par rapport à une ou plusieur(s) caractéristique(s) choisie(s) dans le groupe comprenant courbure de front horizontale, courbure de front verticale, largeur de front, angle fronto-nasal, angle fronto-occipital, diamètre fronto-occipital, diamètre bipariétal, et rayon de courbure horizontal de module occipital, le modèle tridimensionnel se trouve sur une courbe en percentiles qui représente 10% < x%< = 30 % par rapport à une population d'enfants normalement répartie d'âge 0 à 1 an.

6. Procédé selon l'une des revendications 1 à 3, caractérisé en ce par rapport à une ou plusieurs caractéristique(s) choisie(s) dans le groupe comprenant courbure de front horizontale, courbure de front verticale, largeur de front, angle fronto-nasal, angle fronto-occipital, diamètre fronto-occipital, diamètre bipariétal, et rayon de courbure horizontal de module occipital, le modèle tridimensionnel se trouve sur une courbe en percentiles qui représente 30% < x%< 70 % par rapport à une population d'enfants normalement répartie d'âge 0 à 1 an.

7. Procédé selon l'une des revendications 1 à 3, **caractérisé en ce que** par rapport à une ou plusieurs caractéristique(s) choisie(s) dans le groupe comprenant courbure de front horizontale, courbure de front verticale, largeur de front, angle fronto-nasal, angle fronto-occipital, diamètre fronto-occipital, diamètre bipariétal, et rayon de courbure horizontal de module occipital, le modèle tridimensionnel se trouve sur une courbe en percentiles qui représente 70% < x%< 90 % par rapport à une population d'enfants normalement répartie d'âge 0 à 1 an.

8. Procédé selon l'une des revendications 1 à 3, **caractérisé en ce que** par rapport à une ou plusieurs caractéristique(s) choisie(s) dans le groupe comprenant courbure de front horizontale, courbure de front verticale, largeur de front, angle fronto-nasal, angle fronto-occipital, diamètre fronto-occipital, diamètre bipariétal, et rayon de courbure horizontal de module occipital, le modèle tridimensionnel se trouve sur une courbe en percentiles qui représente 90% < x%< 97 % par rapport à une population d'enfants normalement répartie d'âge 0-1 an.

9. Procédé selon l'une des revendications 1 à 8, **caractérisé en ce que** les dimensions externes du modèle tridimensionnel sont réduites de 1 à 5 mm par rapport à une épaisseur d'os d'un crâne de modèle d'enfant humain correspondant.
